(19) Europäisches Patentamt
European Patent Office
Office européen des brevets

(11) Publication number: 0 471 941 A2

# (12) EUROPEAN PATENT APPLICATION

(21) Application number: 91110022.0

(51) Int. Cl.[5]: C07C 43/23, C07C 41/26

(22) Date of filing: 19.06.91

(30) Priority: 16.08.90 US 568138

(43) Date of publication of application:
26.02.92 Bulletin 92/09

(84) Designated Contracting States:
AT BE CH DE DK ES FR GB GR IT LI NL SE

(71) Applicant: AMERICAN CYANAMID COMPANY
1937 West Main Street P.O. Box 60
Stamford Connecticut 06904-0060(US)

(72) Inventor: Green, Kenneth E.
31 Gilbert Avenue
Pearl River, New York 10965(US)

(74) Representative: Wächtershäuser, Günter, Dr.
Tal 29
W-8000 München 2(DE)

## (54) An improved process for the preparation of 5-bromo-2-methoxyresorcinol.

(57) This invention is concerned with an improved process for metallating 2,4,6-tribromoanisole 1 and reacting this intermediate lithio species with various electrophiles for example the trialkoxy borates which an example is trimethylborate, the improvement comprising carrying out the reaction under heterogenous conditions. This product is oxidatively converted to the compound 5-bromo-2-methoxy-resorcinol 2, which is in turn converted to the known 2-methoxy-resorcinol 3. The process and intermediate are useful in the synthesis of platelet activating factor antagonist thiazolium, 3-[[3-[[hydroxy[2-methoxy-3-(tetradecyloxy) phenoxy]phosphinyl]oxy]phenyl]-methyl]-5-methyl-, hydroxide, inner salt CA of U. S. Patent 4983592 (1991).

EP 0 471 941 A2

## BACKGROUND OF THE INVENTION

The metallation of 2,4,6-tribromoanisole is described by Gilman H.; Langham, W.; Moore, F. W. J. Amer. Chem. Soc. 62, 2327(1940) to occur at room temperature in pentane with subsequent reaction of the lithio intermediate with carbon dioxide. Pentane is an attractive solvent because 2,4,6-tribromoanisole is readily soluble. Upon repeating this procedure, only low yields and complex mixtures are obtained.

Attempts to metallate 2,4,6-tribromoanisole at reduced temperatures in solvents such as tetrahydrofuran, where the compounds are soluble, also results in low yields and complex reaction mixtures.

## SUMMARY OF THE INVENTION

This invention is an improved process for preparing 5-bromo-2-methoxyresorcinol by reacting 2,4,6-tribromoanisole with lower alkyl lithium in solvent at about -20°C to about -10°C and subsequently adding an electrophile at about -30°C to about 0°C; the improvement comprising carrying out the reaction under heterogeneous conditions.

## DETAILED DESCRIPTION OF THE INVENTION

This invention is concerned with an improved process for metallating 2,4,6-tribromoanisole 1 with lower alkyl lithium (preferably about 5 equivalents) under heterogenous conditions and reacting this intermediate lithio species with various electrophiles for example the trialkoxy borates of which an example is trimethylborate. This product is oxidatively converted to the compound 5-bromo-2-methoxy-resorcinol 2, which is in turn converted to the Known 2-methoxy-resorcinol 3. (Geisman, T. A. : Moje, W. J. Amer. Chem. Soc. 73, 5765 (1951). More specifically, the invention is the metallation of 1 under heterogeneous conditions. The process and intermediate are useful in the synthesis of platelet activating factor antagonist thiazolium, 3-[[3-[[hydroxy[2-methoxy-3-(tetradecyloxy) phenoxy]phosphinyl]oxy]phenyl]methyl]-5-methyl-, hydroxide, inner salt CA of U. S. Patent 4,983,592 (1991).

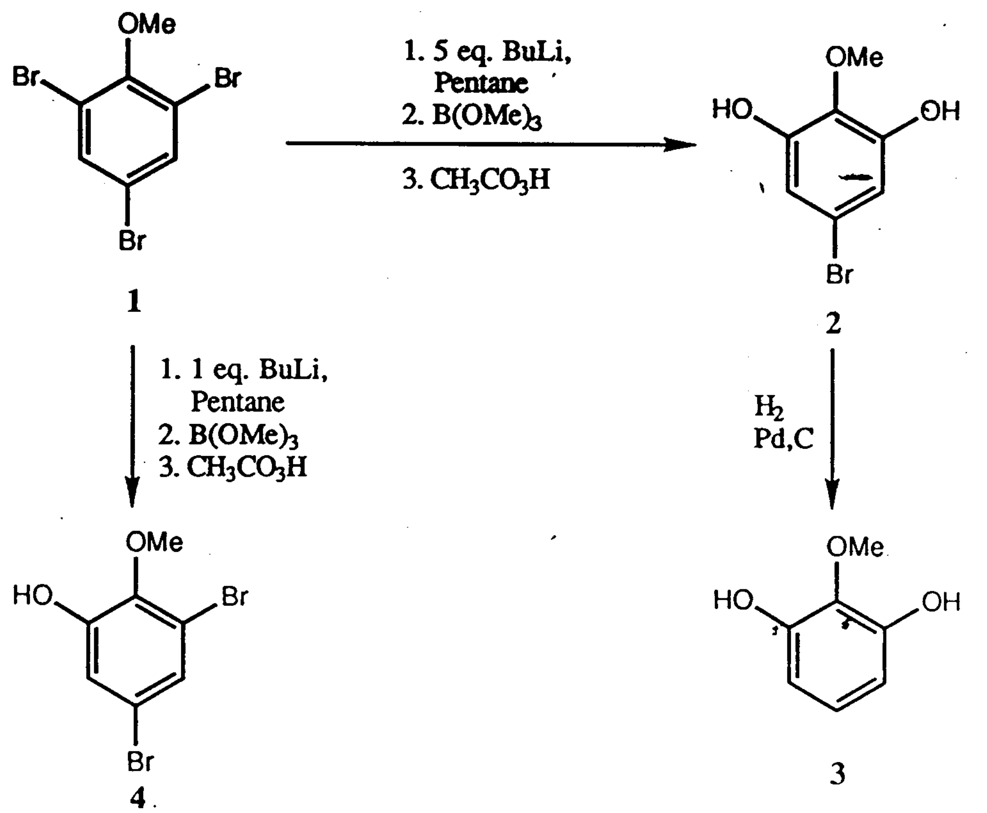


Additionally, by limiting the amount of lower alkyl $C_1$-$C_6$ lithium that is reacted with 1, the novel product 2-methoxy-3,5-dibromophenol 4, a useful intermediate, resulting from reaction of the lithio species with only one equivalent of the electrophile is obtained.

The use of heterogenous conditions is a major improvement over the method reported by Gilman, H. et al., and is unique in that the metallation is occurring on a suspension of 1 at low temperature. Another strategy (Crowther, G.P.; Sundberg, A.M.; Sarpeshkar, A.M. J. Org. Chem. 1984, 49, 4657) involves the direct metallation of anisole and normally occurs at lower temperatures (i.e. -20°C - -78°C) with additives such as N,N,N',N'- tetramethylethylenediamine to effect solubilization of the salt. For materials which may be used in humans, these additives are detrimental in that they are toxic. This compound can be easily converted to 2-methoxy-resorcinol using standard methods. Compound 2 can be used to synthesize 2-methoxy-resorcinol easily and in high yield. The currently reported synthesis (Geisman, et al.) of 2-methoxy-resorcinol is extremely labor intensive and realizes the product in only 3% yield.

The invention will be further described by the following non-limiting examples.

Example 1

5-Bromo-2-Methoxyresorcinol

To a suspension of 2,4,6-tribromoanisole (10g, 30 mmol) in 200 mL of dry pentane at -20°C under an argon atmosphere was added a solution of n-butyllithium in hexanes (93.8 mL of a 1.6 M solution, 150 mmol) over 10 minutes with vigorous mechanical stirring. This suspension was allowed to warm to -10°C over 15 minutes. Upon cooling to -30°C, neat trimethyl borate (15.6g, 150 mmol) was added all at once. The solution was warmed to 0°C over 30 minutes and then cooled to -10°C. A solution of 40% peracetic acid/acetic acid (25 mL) was added over 30 minutes. Upon completion of the addition, the solution was warmed to O°C over 30 minutes and then cooled to -10°C whereupon 25 mL of saturated (aqueous)-$NaHSO_3$ was added dropwise over 30 minutes. Upon warming to room temperature, equal volumes of water and diethyl ether were added. The organic layer was separated, dried over $MgSO_4$, and evaporated to provide a yellow liquid which was dissolved in boiling $CCl_4$ and allowed to cool to room temperature to produce 5-Bromo-2-Methoxybenzene - 1,3 - diol as a white solid (6.0g, 91%): mp 124 - 126°C; $^1$H - NMR (300MHz,$CDCl_3$,ppm) 6.68 (s,2$_{H), 5.38}$ (br s, 2H), 3.86 (s,3H); $^{13}$C NMR (75.5MHz,$CDCl_3$,ppm) 149.47, 138.52, 116.89, 111.76, 61.26. Anal. Calcd for $C_7H_7O_3Br$(219.04): C,38.39; H,3.22; Br,36.48. Found: C,38.34; H,3.27; Br,36.66.

Example 2

2-Methoxy-3,5-dibromophenol

The procedure as described above was followed with the exception that 20 mL (32 mmol) of 1.6 M n-butyllithium and 32.2g (32 mmol) of trimethylborate were used. The product was obtained as a yellow oil which was distilled (bp 120°C/0.24 torr) to give 7.4 g (87%) of 3,5-Dibromo-2-Methoxyphenol as a colorless oil which solidified upon standing: mp 67-68°C; $^1$H-NMR (300 MHz,$CDCl_3$,ppm) 7.22 (d,1 H,J = 2.3Hz), 7.08 (d,1 H,J = 2.3Hz), 5.75 (br s, 1 H), 3.90 (s, 3 H); $^{13}$C NMR (75.5 MHz, $CDCl_3$,ppm) 150.49, 143.91, 126.94, 118.43, 117.61, 116.33, 61.20. Anal. Calcd for $C_7H_6Br_2O_2$ (281.93): C,29.82; H,2.14; Br,56.68. Found: C,29.89; H,2.18; Br,56.93.

Example 3

2-Methoxyresorcinol

To a solution of 5-Bromo-2-Methoxybenzene - 1,3 - diol (5g,23 mmol) in ethyl acetate (30 mL) was added 5% Pd/C (500 mg) and this suspension was hydrogenated on a Paar shaker to produce 2 - Methoxybenzene - 1,3 - diol quantitatively (3.2g) as a white solid after filtration of the suspension and evaporation of the filtrate: mp 85-87°C (lit mp 84.5-85°C)[1]; $^1$H-NMR(300 MHz,,$CDCl_3$,ppm) 6.88 (t, 1 H,J = 8.3Hz), 6.51 (d,2H,J = 8.2Hz), 5.31(br s, 2 H), 3.88 (s, 3 H); $^{13}$C NMR (75.5 MHz, $CDCl_3$, ppm) 148.97, 134.63, 124,80, 108.19, 61.17. Anal. Calcd for $C_7H_8O_3$(140.14): C, 60.00; H, 5.75. Found: C,59.76; H,5.72.

**Claims**

**1.** In a process for preparing 5-bromo-2-methoxyresorcinol by reacting 2,4,6-tribromoanisole with lower alkyl lithium in solvent at about -20°C to about -10°C and subsequently adding an electrophile at about -30°C to about 0°C, the improvement comprising carrying out the reaction under heterogeneous

conditions.

2. A process according to claim 1 wherein the n-lower alkyl lithium is n-butyl lithium, the solvent is pentane and the electrophile is a trimethylborate.

3. The compound 2-Methoxy-3,5-dibromophenol.